# FASCICULE DE BREVET EUROPEEN

(11) **EP 2 521 499 B1**
(45) Date de publication et mention de la délivrance du brevet: **16.12.2015**
(21) Numéro de dépôt: 11704642.5
(22) Date de dépôt: 04.01.2011
(51) Int. Cl.: A61B 17/66

(54) **DISTRACTEUR MANDIBULAIRE INTRAORAL SUR MESURE**
PERSONALISIERTER INTRAORALER KIEFERDISTRAKTOR
CUSTOMISED INTRAORAL JAW DISTRACTOR

(30) Priorité: 04.01.2010 FR 1000014
(43) Date de publication de la demande: 14.11.2012
(73) Titulaire: OBL (Société Anonyme), 92320 Chatillon (FR)
(72) Inventeur: DUBOIS, Guillaume, F-92170 Vanves (FR)
(74) Mandataire: Blaseby, Matthew Peter
(86) Numéro de dépôt international: PCT/FR2011/000004
(87) Numéro de publication internationale: WO 2011/083260

(56) Documents cités:
- EP-A1- 2 133 033
- FR-A1- 2 829 684
- JP-A- 11 262 491
- US-B1- 6 972 020

## Description

### DOMAINE TECHNIQUE DE L'INVENTION

La présente invention concerne un distracteur mandibulaire intraoral sur mesure pour la chirurgie maxillo-faciale, indiqué notamment pour les reconstructions mandibulaires, apte à permettre une distraction selon un mode quasi continu.

### ARRIÈRE-PLAN TECHNOLOGIQUE DE L'INVENTION

Le remplacement d'un fragment osseux manquant à la mâchoire inférieure, ou réparateur de celle-ci, par exemple à la suite d'une résection tumorale ou d'un traumatisme important, ou plus simplement la cicatrisation d'un os à la suite d'une ostéotomie pratiquée en vue de modifier l'anatomie osseuse, fait appel à différents types de techniques opératoires.

L'une de ces techniques, la distraction ostéogénique, qui est un procédé chirurgical utilisé pour reconstruire les déficiences ou défauts osseux ou pour allonger les os du corps humain, consiste à placer un appareillage mécanique appelé distracteur dont le rôle est de tirer sur le cal osseux résultant de l'ostéotomie pour l'allonger d'une longueur correspondant à la perte osseuse.

Quel que soit l'organe concerné, ce procédé est effectué en plusieurs étapes qui peuvent être décomposées de la manière suivante. On réalise tout d'abord un geste chirurgical qui consiste principalement en une ostéotomie sous-périostée afin de fracturer l'os à reconstruire et donc de le diviser en au moins deux segments. Après une période de latence, ces segments sont progressivement éloignés l'un de l'autre, ou les uns des autres, à l'aide d'un distracteur. Une étape de maintien, permettant la consolidation osseuse, précède bien évidemment la dépose dudit distracteur.

Diverses techniques de distraction ostéogénique sont déjà utilisées avec succès dans les pertes de substances interruptrices de la mandibule, du maxillaire ou de l'os malaire, en particulier dans les traumatismes balistiques (par exemple à la suite de tentatives de suicide par arme à feu) de la face. La distraction ostéogénique permet une reconstruction de l'os, de la gencive et des tissus mous en regard.

Divers distracteurs correspondants à ces diverses techniques de reconstruction des pertes osseuses et des parties molles de l'étage inférieur ou de l'étage moyen de la face sont donc déjà connus de l'art antérieur.

Il existe des distracteurs osseux externes, dont l'essentiel des mécanismes est extérieur à la cavité buccale, et des distracteurs intraoraux qui sont suffisamment miniaturisés pour être en totalité intégrés dans la bouche du patient dont la mâchoire est à réparer.

Un exemple de distracteur osseux externe est divulgué dans la demande de brevet FR-2.829.684 déposée par la société OBL et publiée le 21 mars 2003. Ce distracteur externe comprend un rail cylindrique fileté reliant deux moyens d'ancrage situés de part et d'autre du cal osseux et un chariot mobile qui est guidé à coulissement sur le rail et qui est fixé sur le segment osseux à distracter. Le chariot mobile est équipé d'un écrou dont le filetage est complémentaire de celui du rail, de sorte qu'il puisse coopérer avec ce dernier, et de deux pièces percées chacune d'un alésage dont la section est complémentaire de celle du rail. Le déplacement du chariot le long du rail est commandé par la rotation de l'écrou, les alésages des deux pièces percées constituant les moyens de guidage du chariot au cours de son coulissement sur le rail.

La demande de brevet EP-0.770.359 déposée par la société Medicon, publiée le 2 mai 1997, décrit un système de distraction mandibulaire mettant en oeuvre une première technique. Un vérin à vis miniature, dont les deux extrémités sont fixées de part et d'autre du cal ostéogénique, exerce une traction sur celui-ci, de manière à assurer la néoformation d'un tissu osseux de remplacement à son emplacement. L'ensemble du dispositif est entièrement contenu dans la cavité buccale et la vis peut être manoeuvrée de l'extérieur par un outil de type "tournevis".

Mais l'appareil demeure encombrant, notamment la partie télescopique, et il est impossible d'en placer plusieurs du même côté de la mandibule.

Un dispositif un peu plus compact, constitué par un double vérin miniature à vis agissant sur deux pièces symétriques, lesquelles sont solidaires des points d'ancrage et sont guidées par deux tiges parallèles, est décrit dans la demande de brevet internationale WO 98/16163 au nom M. Chin, publiée le 23 avril 1998. L'ensemble est en quelque sorte un rectangle de longueur variable, dont l'extension est réglée par le petit vérin.

Le dispositif Chin est apte à exercer des efforts importants et est particulièrement adapté à traiter la branche montante de la mandibule, mais sa réalisation mécanique est assez complexe.

La demande de brevet internationale WO 98/09577 au nom de M. Mommaerts, publiée le 12 mars 1998, concerne un dispositif toujours basé sur le même principe, mais d'un mode de réalisation encore différent. L'extrémité postérieure du système à vis télescopique prend appui directement sur un insert placé dans la branche montante de la mandibule, au lieu d'être fixée sur une barrette perforée classique en implantologie. Le système est donc plus simple et plus compact, mais il ne permet de traiter que des zones très en arrière de la mâchoire.

La demande de brevet JP-11/262491 au nom de Nagoya Screw MFG Co Ltd, publiée le 28 septembre 1999, décrit un distracteur intraoral comprenant un rail étroit dont un petit chant longitudinal est doté d'une crémaillère, ledit rail, monté fixement dans l'os de la mâchoire du patient, entre deux points d'ancrage, recevant un chariot mobile qui est fixé sur le segment osseux à distracter et qui est équipé d'une vis sans fin dont le filetage coopère avec la crémaillère du rail. Le déplacement du chariot le long du rail est commandé par la rotation de la vis sans fin.

Les avantages de ces distracteurs mandibulaires résident dans le fait qu'ils sont d'une application intraorale simple et semblable aux méthodes d'ostéosynthèses, que leur activation est aisée et, par conséquent, qu'ils sont acceptés sans problème par les patients.

Toutefois une lacune demeure dans leurs indications thérapeutiques, car il ressort de l'état de la technique tel que décrit ci-dessus qu'aucun de tels distracteurs mandibulaires ne procure une continuité dans l'effort ou dans la vitesse de distraction. Tous ces appareils fonctionnent de façon discontinue, à savoir que l'on tire périodiquement sur l'os à reconstruire, de sorte à le déplacer subitement de quelques dixièmes de millimètre à chaque fois, la capacité spontanée de l'os à se régénérer comblant dans les heures suivantes l'espace induit par la traction du distracteur, puis que l'on renouvelle l'opération de distraction en déplaçant subitement à nouveau de quelques dixièmes de millimètre l'os à reconstruire (selon un protocole de référence, accepté par tous les praticiens, la traction sur l'os à reconstruire est classiquement effectuée de manière quotidienne ou parfois biquotidienne et cette traction est de l'ordre de 0,8 mm à 1 mm par jour).

Les distracteurs connus ont en outre pour la plupart un, voire deux inconvénients : celui d'être très encombrants et celui de ne pas permettre directement la reconstruction de l'os selon une trajectoire spécifique au patient traité, notamment lorsque tout ou partie de cette trajectoire est courbe.

Le distracteur mandibulaire intraoral selon l'invention permet de pallier les inconvénients précités : la force qu'il applique, tendant à déplacer l'os à reconstruire, est quasi continue ; il est compact, ce qui prend toute son importance si l'on considère le caractère intraoral d'un tel dispositif mécanique ; en outre, il peut être construit à l'image du patient, être en quelque sorte spécifique à chaque patient, en respectant les parties rectilignes et les parties courbes de ce que devrait idéalement respecter l'os après sa reconstruction.

Ainsi, la géométrie du distracteur selon l'invention pourra être définie par informatique en pré-opératoire, en prenant en compte : la perte de substance à la suite de l'opération ou du traumatisme, la localisation de cette perte de substance, la localisation et le volume de l'os restant exploitable, et enfin l'image que l'on souhaiterait redonner au patient.

### DESCRIPTION GÉNÉRALE DE L'INVENTION

La présente invention a donc pour premier objet un distracteur mandibulaire intraoral sur mesure, destiné à la reconstruction par distraction ostéogénique d'un segment osseux manquant ou déficient à la suite d'une résection tumorale, d'un traumatisme, d'une malformation osseuse ou d'une hypoplasie osseuse.

De manière connue, en ce qu'il doit provoquer une stimulation osseuse par la contrainte en traction, un tel distracteur comporte deux moyens d'ancrage fixés dans le corps mandibulaire en des points d'ancrage fixes, situés de part et d'autre du cal osseux.

L'invention a plus précisément pour objet un distracteur mandibulaire intraoral de ce type dont la caractéristique essentielle est qu'il comprend :
a) un rail cylindrique fileté reliant les deux moyens d'ancrage,
b) un chariot mobile guidé à coulissement sur le rail et fixé sur le segment osseux à distracter, ledit chariot mobile étant équipé d'une vis sans fin dont le filetage est complémentaire de celui du rail et coopère avec ce dernier,
c) un axe solidaire de la vis sans fin et commandant la rotation de celle-ci, ledit axe étant sensiblement parallèle au rail et étant supporté par deux paliers agencés sur le chariot mobile, de part et d'autre de la vis sans fin,
d) au moins une pièce élastique qui est traversée par ledit axe et qui vient en appui d'une part contre une extrémité de la vis sans fin et d'autre part contre la paroi interne du palier qui fait face à ladite extrémité, la distance séparant les parois internes des deux paliers du chariot mobile étant égale à la longueur de la vis sans fin augmentée de la hauteur de la pièce élastique lorsque celle-ci est au repos, détendue.

Idéalement, la pièce élastique est soit une rondelle du type Grower, soit une rondelle tronconique dite Belleville, soit un empilement de telles rondelles tronconiques disposées en opposition. Cependant, et sans que cette énumération soit limitative, la pièce élastique peut être aussi tout autre élément élastique tel qu'un ressort, un diaphragme ou un cylindre compressible, par exemple en élastomère.

De façon à permettre au praticien ou au patient lui-même une activation aisée du distracteur, l'axe commandant la rotation de la vis sans fin est équipé d'une tête de manoeuvre logée dans un évidement pratiqué dans la paroi externe du palier contre lequel la pièce élastique vient en appui.

Selon diverses variantes envisageables pour parvenir à une telle activation, la tête équipant l'axe de commande de la rotation de la vis sans fin présente une fente, ou deux fentes disposées en croix, ou un trou borgne à six pans permettant la manoeuvre en rotation de ladite tête au moyen d'un tournevis ou d'une clé Allen.

Afin de garantir une parfaite fixation et une parfaite utilisation du distracteur selon l'invention, chacun de ses deux moyens d'ancrage et son chariot mobile sont fixés, respectivement dans le corps mandibulaire et dans le segment osseux à distracter, au moyen d'au moins deux points de fixation.

Selon diverses formes de réalisation envisageables, les moyens de fixation du chariot mobile dans le segment osseux à distracter ont la forme d'un I, d'un Y ou d'une croix de Saint-André.

Idéalement, pour permettre un parfait guidage à coulissement du chariot mobile, le rail cylindrique fileté du distracteur selon l'invention présente deux méplats diamétralement opposés, le chariot mobile guidé à coulissement sur ledit rail ayant la forme d'une cage dont les deux parois parallèles à l'axe longitudinal du rail, réunies à leurs deux extrémités par l'un et l'autre des deux paliers supportant l'axe de la vis sans fin, sont espacées d'une distance très légèrement supérieure à l'épaisseur dudit rail prise entre ses deux méplats. Dans le même but, le rail cylindrique fileté peut présenter un troisième méplat en regard du fond du chariot mobile.

Dans une première forme de réalisation du distracteur selon l'invention, son rail est rectiligne.

Dans une seconde forme de réalisation du distracteur selon l'invention, son rail forme au moins une courbe.

Dans ce second cas, la ou les différentes courbures du rail appartiennent à un plan unique, lequel plan est perpendiculaire aux méplats du rail afin de préserver le filetage au voisinage des fibres neutres dudit rail.

Quels que soient les modes de réalisation du distracteur selon l'invention, chacun de ses éléments constitutifs est avantageusement réalisé en un matériau biocompatible, par exemple en titane ou en alliage de titane.

La présente invention a également pour objet une utilisation bien spécifique du distracteur répondant aux caractéristiques précitées, pour une distraction selon un mode quasi continu, cette utilisation étant remarquable par le fait que l'on commande la rotation de l'axe solidaire de la vis sans fin de sorte à comprimer à nouveau la pièce élastique dès que l'extrémité de la vis sans fin qui n'est pas en appui contre ladite pièce élastique revient au contact de la paroi interne du palier qui fait face à ladite extrémité.

Ces quelques spécifications essentielles rendent évidentes pour l'homme de métier les avantages supplémentaires apportés par le distracteur mandibulaire intraoral selon l'invention par rapport à l'état antérieur de la technique.

Les spécifications détaillées de l'invention sont données dans la description qui suit en liaison avec les dessins ciannexés. Il est à noter que ces dessins n'ont d'autre but que celui d'illustrer le texte de la description et qu'ils ne constituent donc en aucune sorte une limitation de la portée de l'invention.

### BRÈVE DESCRIPTION DES DESSINS

La Figure 1 est une vue en perspective de l'avant d'un distracteur mandibulaire intraoral sur mesure selon l'invention, réalisé selon son premier mode dans lequel le rail cylindrique fileté est rectiligne.
La Figure 2 est une vue en perspective de l'avant d'un distracteur mandibulaire intraoral sur mesure selon l'invention, réalisé selon son second mode dans lequel le rail cylindrique fileté est courbe.
La Figure 3 est une vue en perspective de l'arrière du distracteur de la Figure 2
La Figure 4 représente, de face et de l'avant, une vue plus détaillée du chariot mobile guidé à coulissement sur le rail cylindrique fileté.
La Figure 5 est une vue en perspective du chariot mobile représenté à la Figure 4.

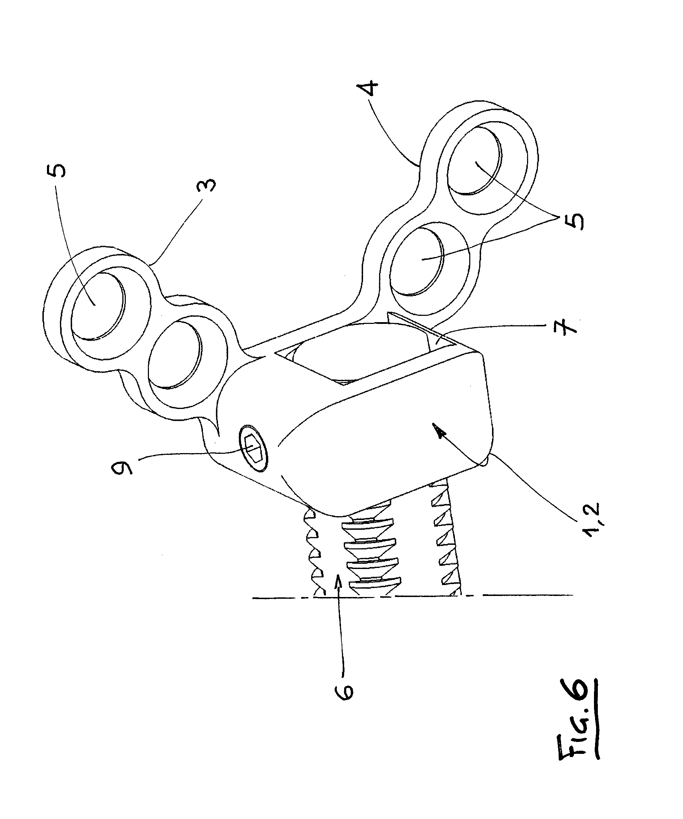

La Figure 6 est une vue en perspective d'un des deux moyens d'ancrage fixes du distracteur selon l'invention, ledit moyen d'ancrage étant apte à être fixé dans le corps mandibulaire, d'un côté ou de l'autre du cal osseux.

### DESCRIPTION DES MODES DE RÉALISATION PRÉFÉRÉS DE L'INVENTION

Le distracteur mandibulaire intraoral sur mesure réalisé conformément à l'invention comporte de façon connue deux moyens d'ancrage, respectivement gauche 1 et droit 2, fixés dans le corps mandibulaire du patient en des points d'ancrage déterminés par l'opérateur et situés de part et d'autre du cal osseux.

Afin de constituer des points d'ancrage fixes, chacun de ces deux moyens d'ancrage 1 et 2 comporte de préférence deux pattes 3 et 4 formant par exemple entre elles un angle de l'ordre de 90 à 120°, chaque patte étant par exemple dotée de deux orifices 5 qui seront traversés par des vis ou des broches (non représentées), lesquelles garantiront ainsi la fixation desdits moyens d'ancrage dans les segments osseux immobiles du corps mandibulaire.

Un rail cylindrique fileté 6, reliant les deux moyens d'ancrage 1 et 2, constitue la base du distracteur selon l'invention, en ce sens qu'il va permettre de définir très exactement le trajet osseux de la reconstruction.

Chacune des deux extrémités du rail 6 vient à cette fin se loger dans un évidement 7 pratiqué dans la paroi intérieure 8 de chacun des deux moyens d'ancrage 1, 2 qui est opposée aux pattes 3, 4.

Chaque extrémité du rail 6 est ensuite fixée dans ledit évidement 7 qu'elle pénètre, et ce à l'aide d'une vis 9 traversant un trou fileté percé entre le sommet (ou la base, ou encore une paroi longitudinale) et le logement 7 de chacun des deux moyens d'ancrage 1, 2.

Un chariot 10, mobile à coulissement sur le rail 6, et guidé par ce dernier, est fixé sur le segment osseux à distracter.

Pour permettre son coulissement, le chariot mobile 10 est équipé d'une vis sans fin 11 dont le filetage est complémentaire de celui du rail 6 et coopère avec ce dernier. Le pas de l'ensemble « vis sans fin/filetage du rail » est choisi en correspondance avec les valeurs de déplacement utilisées de façon classique pour la distraction.

La vis sans fin 11 est percée d'un trou traversant qui s'étend selon l'axe longitudinal de ladite vis, trou traversant dans lequel est logé un axe central 12 qui déborde de part et d'autre de la vis sans fin 11 et dont le rôle est de commander la rotation de ladite vis. L'axe 12 est rendu solidaire de la vis sans fin 11, soit en étant monté serré, soit par soudure, soit encore par collage, par exemple au moyen d'une colle cyanocrylate.

L'axe 12 ainsi solidaire de la vis sans fin 11 est donc sensiblement parallèle au rail fixe 6 et il est supporté par deux paliers, respectivement gauche 13 et droit 14, agencés sur le chariot mobile 10 de part et d'autre de la vis sans fin.

Enfin, au moins une pièce élastique 15 traversée par l'axe 12 vient en appui, d'une part, contre une extrémité 16 de la vis sans fin 11 et, d'autre part, contre la paroi interne 18 du palier 14 qui fait face à ladite extrémité 16, la distance séparant les parois internes, respectivement 17 et 18, des deux paliers 13 et 14 du chariot mobile 10 étant égale à la longueur de la vis sans fin 11 augmentée de la hauteur de la pièce élastique 15 lorsque celle-ci est au repos, c'est-à-dire détendue.

L'axe central 12 déborde de part et d'autre de la vis sans fin 11 pour pouvoir être supporté par les paliers 13 et 14, respectivement dans un logement percé dans la paroi interne 17 du palier 13 et dans un guide percé dans la paroi interne 18 du palier 14, ce guide traversant ledit palier 14 et débouchant dans sa paroi externe 19.

Un évidement 20 d'un diamètre supérieur à celui du guide est pratiqué dans ladite paroi externe 19 et, de façon complémentaire, l'extrémité de l'axe central 12 de la vis sans fin 11 est équipée d'une tête 21 qui vient se loger dans l'évidement 20.

De tout ce qui précède, on comprend que l'axe central 12 est rendu solidaire de la vis sans fin 11 après que ladite vis et la pièce élastique 15 ont été mises en place côte à côte entre les deux paliers 13 et 14, contre leurs faces internes, respectivement 17 et 18, et après que ledit axe a été monté traversant le guide du palier 14, la pièce élastique 15 puis le trou longitudinal de la vis sans fin 11, et enfin logé dans le palier 13.

Les deux extrémités de l'axe central 12 peuvent tourner librement dans les paliers 13 et 14 qui supportent ledit axe central.

Par construction, la profondeur du logement percé dans la paroi interne 17 du palier 13 est au moins égale à la longueur de la partie de l'axe central 12 qui déborde de la vis sans fin 11 et qui est appelée à pénétrer ledit logement, et la longueur de cette partie en saillie de l'axe central 12 est supérieure à la perte d'épaisseur maximale que la pièce élastique peut connaître au cours de sa compression.

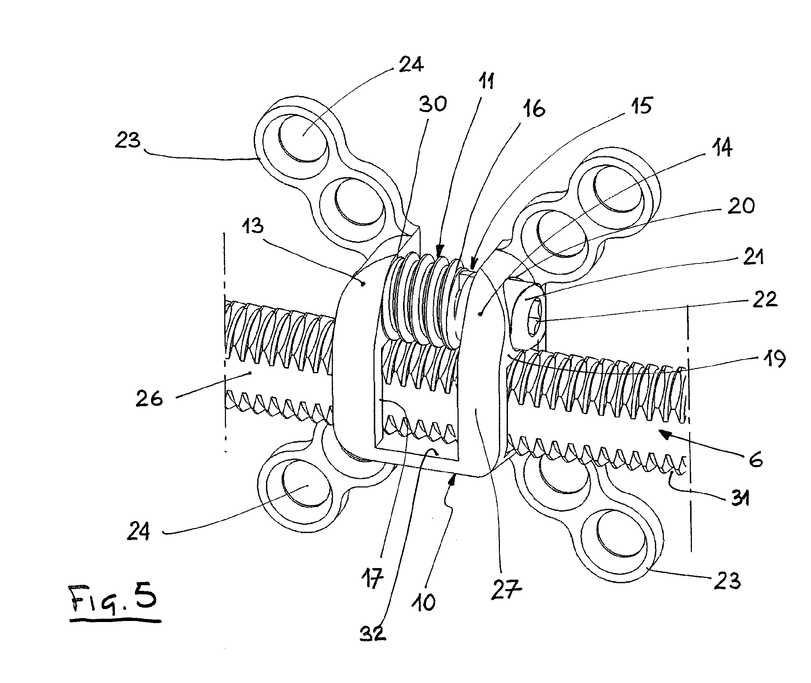

La tête 21 de l'axe central 12 est en outre pourvue de moyens 22 permettant sa manoeuvre en rotation, par exemple sous la forme d'une fente ou de deux fentes disposées en croix, aptes à coopérer avec un tournevis, ou encore sous la forme d'un trou borgne à six pans, ainsi qu'il est représenté à la figure 5, apte à coopérer avec une clé Allen.

Dès lors que l'axe central 12 et la vis sans fin 11 ont été rendus solidaires, il est clair que la manoeuvre en rotation de la tête 21 de l'axe 12 au moyen d'un tournevis ou d'une clé Allen commande la rotation de la vis sans fin 11 dans son ensemble.

La pièce élastique 15 qui équipe le chariot mobile 10 est choisie pour se comporter dans le sens axial comme un ressort de faible encombrement et de raideur relative. Ainsi, la force nécessaire pour comprimer cette pièce élastique est en effet choisie pour être légèrement inférieure à celle voulue pour éloigner le segment osseux devant être distracté du segment osseux immobile dont il provient après fracture.

La pièce élastique 15 peut donc être une rondelle élastique, par exemple soit une rondelle tronconique, encore dénommée rondelle Belleville, soit un empilement de telles rondelles tronconiques disposées en opposition, soit et de préférence une rondelle du type Grower, c'est-à-dire une rondelle annulaire qui présente une coupure et qui, au niveau de cette coupure, est fortement déformée dans le sens axial.

C'est ainsi une rondelle Grower qui a été représentée à titre d'exemple dans tous les dessins annexés à la présente description. Toutefois, la pièce élastique 15 peut également être réalisée selon une forme techniquement équivalente à une rondelle, par exemple un ressort, un diaphragme, ou encore un cylindre compressible, par exemple en élastomère.

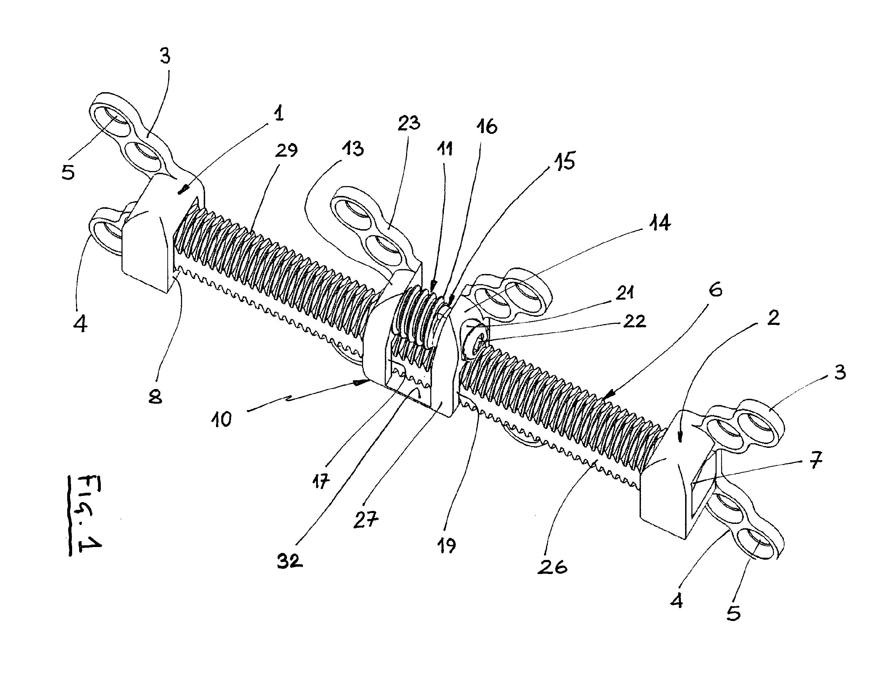

Enfin, le chariot mobile 10 est doté de moyens de fixation dans le segment osseux à distracter. Ces moyens sont par exemple des ailes 23 débordant de part et d'autre dudit chariot, solidaires de celui-ci, et ayant par exemple la forme d'un I, d'un Y ou encore d'une croix de Saint-André comme cela est représenté sur les figures 1 à 5.

Chaque aile 23 de la croix de Saint-André est alors avantageusement percée de deux orifices 24 pour le passage de vis ou de broches (non représentées) qui vont assurer la fixation du chariot mobile 10 dans le segment osseux à distracter.

De tout ce qui précède, on comprend que, en commandant la rotation de la vis sans fin 11 à l'aide d'un tournevis ou d'une clé Allen manoeuvrant la tête 21 de l'axe central 12 solidaire de ladite vis sans fin, on peut comprimer la rondelle élastique 15 sans déplacer le chariot 10, ou du moins en ne le déplaçant qu'insensiblement, donc sans écarter par cette manoeuvre le segment osseux devant être distracté du segment osseux immobile.

Dans l'exemple représenté, si cette manoeuvre en rotation est effectuée dans le sens inverse de celui des aiguilles d'une montre, la rondelle élastique 15 va être progressivement comprimée, la vis sans fin 11 faisant alors pression par son extrémité 16 sur ladite rondelle élastique en même temps que l'autre extrémité 30 de la vis sans fin s'éloigne de la paroi interne 17 du palier 13.

Au cours de cette déformation, la rondelle élastique va accumuler une certaine quantité d'énergie, égale au travail effectué par la force qui a permis de la comprimer, et l'emmagasiner sous forme d'énergie potentielle élastique.

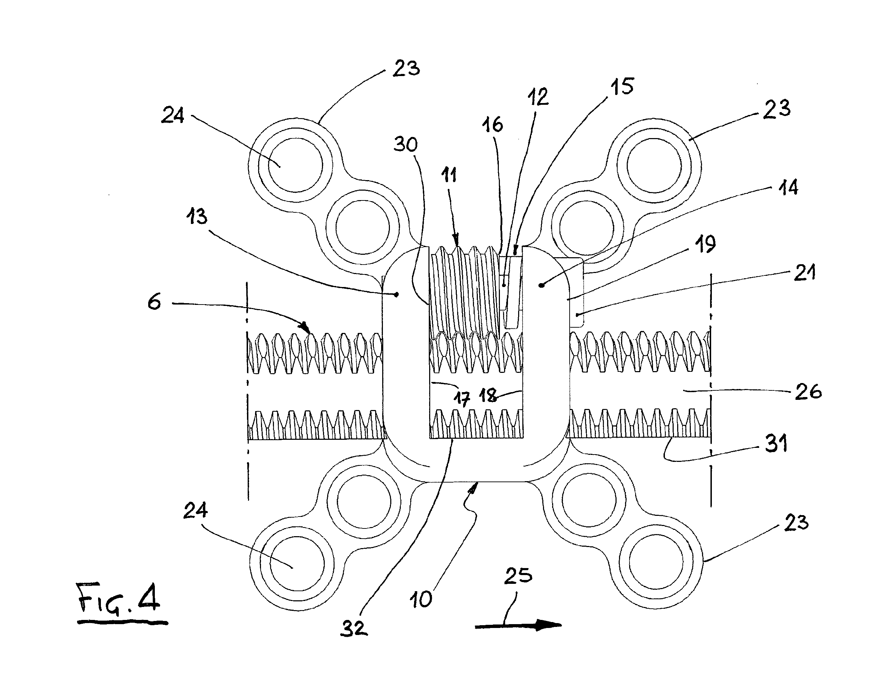

En pratique, lors de la rotation précitée de la vis sans fin, la rondelle 15 se comprime à la manière d'un ressort mais le segment osseux à distracter n'avance pas, ou du moins n'avance que de façon insensible. Dans les heures qui suivent, l'énergie potentielle élastique emmagasinée par la rondelle 15 est restituée, ce qui a pour effet que la rondelle tend alors à reprendre doucement sa forme initiale de repos, c'est-à-dire détendue, jusqu'à ce que ladite énergie potentielle élastique devienne nulle. Autrement dit, la rondelle 15 se détend en s'appuyant sur l'extrémité 16 de la vis sans fin, qui est immobilisée, et elle repousse ainsi le palier 14 ; le chariot 10 sera alors déplacé très progressivement du même mouvement, de la gauche vers la droite comme l'indique la flèche 25 à la figure 4, entraînant toujours dans le même mouvement progressif le segment osseux à distracter. La distraction s'effectue ainsi de façon quasi continue, à la différence de ce que permettent les distracteurs connus jusqu'ici. Or, d'après les recherches les plus récentes, il semble que l'ostéogénèse serait d'autant plus performante que la distraction serait menée de la manière la plus continue qui soit. Le distracteur selon l'invention permet donc d'améliorer le taux de succès des traitements tout en réduisant le temps nécessaire à ceux-ci.

Afin d'obtenir une distraction selon un mode continu ou quasi continu, le praticien (ou le patient lui-même) fera donc en sorte que, dès que l'extrémité 30 de la vis sans fin 11 qui n'est pas en appui contre la rondelle élastique 15 viendra (ou reviendra) au contact de la paroi interne 17 du palier 13 qui fait face à ladite extrémité 30, il renouvelle la précédente opération au cours de laquelle, par la manoeuvre de la tête 21 de l'axe central 12 de ladite vis sans fin, il avait comprimé la rondelle élastique 15. Puisqu'il y aura ainsi continuité dans la distraction, le praticien pourra agir en pratique au quotidien, voire deux fois par jour, en comprimant à chaque fois la rondelle 15, en réduisant ainsi son épaisseur de plusieurs dixièmes de millimètre à deux millimètres, et en écartant donc d'autant, mais cette fois avec progressivité, le segment osseux à distracter relativement au segment osseux immobile.

Le chariot mobile 10 du distracteur selon l'invention devra être guidé de façon parfaite par le rail 6 aussi longtemps que la distraction le nécessitera.

Dans ce but, la tige filetée standard à partir de laquelle le rail 6 est fabriqué subira un usinage particulier consistant en la prévision, sur toute la longueur de ladite tige, de deux méplats 26 diamétralement opposés, sensiblement parallèles aux pattes 3 des moyens d'ancrage 1 et 2.

Selon une construction complémentaire préférentielle, le chariot mobile 10 aura alors la forme d'une cage dont les deux parois latérales, respectivement avant 27 et arrière 28, parallèles à l'axe longitudinal du rail 6 et aux méplats 26 de celui-ci, seront réunies à leurs deux extrémités par l'un et l'autre des deux paliers 13 et 14, lesdites deux parois 27 et 28 étant en outre espacées d'une distance très légèrement supérieure à l'épaisseur du rail 6 prise entre ses deux méplats.

Dans une première variante de réalisation, la plus ordinaire, le rail 6 sera rectiligne, ainsi qu'il a été représenté à la Figure 1.

Toutefois, la prévision des deux méplats 26 permettra la réalisation d'autres variantes infiniment plus complexes dans lesquelles le rail 6 formera une ou plusieurs courbes, imposant ainsi le déplacement idéal recherché par le praticien au segment osseux à distracter.

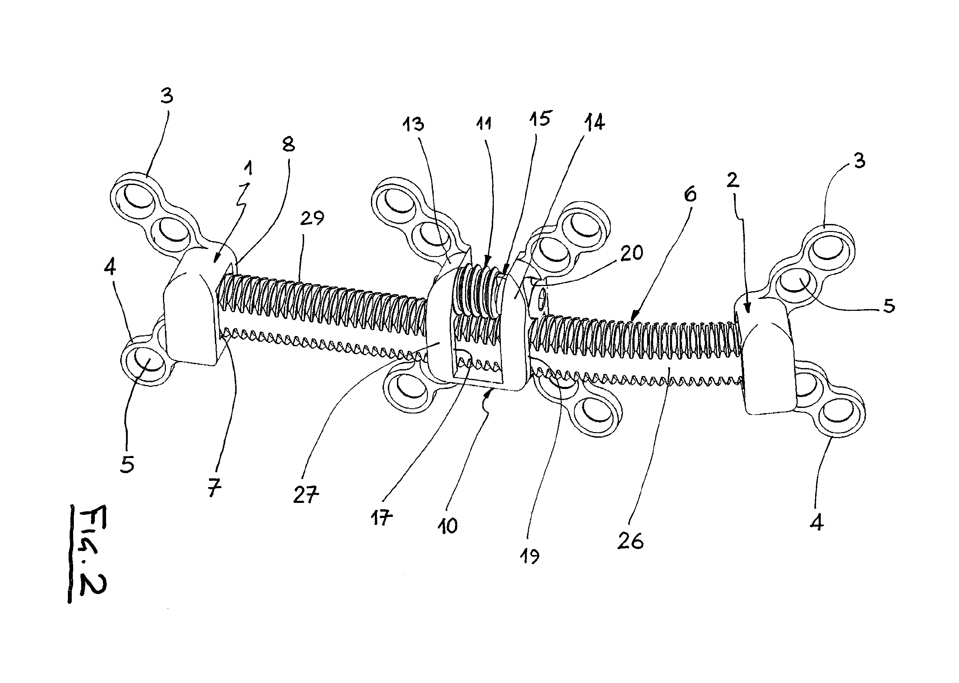

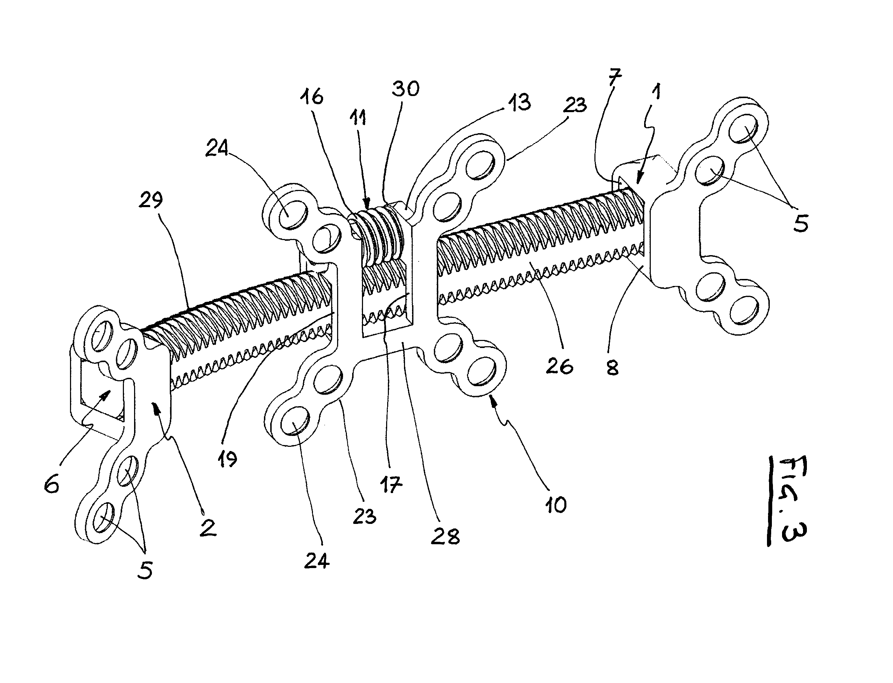

Un exemple de rail à une courbe a été représenté aux Figures 2 et 3.

On conçoit cependant que le rail puisse être courbé à façon et comprendre plusieurs courbures, globalement de mêmes concavités ou de concavités inverses, et qu'il épouse ainsi au plus proche la forme intérieure de la mandibule du patient, la seule limite de ces différentes courbures étant qu'elles appartiennent à un plan unique, repérable en ce qu'il est perpendiculaire aux méplats 26 du rail, et ce afin de préserver le filetage d'origine de la tige au voisinage des fibres neutres 29 qui ne subissent ni extension, ni compression lors de la réalisation des courbes. Les caractéristiques nominales du profil des filets et du pas de la tige filetée seront ainsi conservées et la coopération parfaite du filetage du rail avec la vis sans fin ne sera nullement affectée, quelles que soient la quantité et la forme des courbures dudit rail.

Le distracteur selon l'invention peut donc être spécifique à chaque patient et être réalisé sur mesure à partir de données obtenues par informatique au stade préopératoire.

Un autre avantage d'un tel distracteur est sa grande compacité. Son épaisseur hors-tout, fixations incluses, est en effet de six millimètres. Cette particularité est à l'évidence de première importance si l'on prend simultanément en compte le caractère intraoral de ce dispositif.

Du fait toujours de son caractère intraoral, chacun des éléments constitutifs du distracteur selon l'invention, y compris chacune de ses vis de fixation, sera réalisé en un matériau biocompatible, par exemple en titane ou en alliage de titane.

Selon encore une autre variante de réalisation, et dans le but d'améliorer davantage la stabilité du mécanisme, il est aisé de concevoir un distracteur conforme à celui décrit précédemment et dans lequel le rail fileté 6 présente un troisième méplat qui s'étend sur toute la longueur de la partie inférieure 31 du rail, c'est-à-dire en regard du fond 32 de la cage constituant le chariot mobile 10. Le chariot mobile 10 sera ainsi encore mieux guidé le long du rail 6 puisque trois de ses faces internes, celles présentes sur ses deux parois latérales respectivement avant 27 et arrière 28 et celle présente sur son fond 32, seront à proximité immédiate des trois méplats 26 et 31 prévus sur le rail 6. Dans cette variante, mises à part les zones de rattachement entre les méplats, c'est alors essentiellement la partie supérieure du rail qui laissera apparaître le filetage indispensable à la manoeuvre de la vis sans fin 11 et, par suite, indispensable au déplacement du chariot 10.

Comme il va de soi, l'invention ne se limite pas aux seuls modes d'exécution préférentiels décrits ci-dessus.

Elle embrasse au contraire toutes les variantes possibles de réalisation pour autant que ces dernières demeurent dans le cadre défini par les revendications ci-après.

## Revendications

1. Distracteur mandibulaire intraoral sur mesure, destiné à la reconstruction par distraction ostéogénique d'un segment osseux manquant ou déficient à la suite d'une résection tumorale, d'un traumatisme, d'une malformation osseuse ou d'une hypoplasie osseuse, ledit distracteur, du type comportant deux moyens d'ancrage (1, 2) fixés dans le corps mandibulaire en des points d'ancrage fixes, situés de part et d'autre du cal osseux, étant **caractérisé en ce qu'**il comprend :
a) un rail cylindrique fileté (6) reliant les deux moyens d'ancrage (1, 2),
b) un chariot mobile (10) guidé à coulissement sur le rail (6) et fixé sur le segment osseux à distracter, ledit chariot mobile étant équipé d'une vis sans fin (11) dont le filetage est complémentaire de celui du rail et coopère avec ce dernier,
c) un axe (12) solidaire de la vis sans fin (11) et commandant la rotation de celle-ci, ledit axe étant sensiblement parallèle au rail (6) et étant supporté par deux paliers (13, 14) agencés sur le chariot mobile (10), de part et d'autre de la vis sans fin,
d) au moins une pièce élastique (15) qui est traversée par ledit axe (12) et qui vient en appui d'une part contre une extrémité (16) de la vis sans fin (11) et d'autre part contre la paroi interne (18) du palier (14) qui fait face à ladite extrémité (16), la distance séparant les parois internes (17, 18) des deux paliers (13, 14) du chariot mobile (10) étant égale à la longueur de la vis sans fin (11) augmentée de la hauteur de la pièce élastique (15) lorsque celle-ci est au repos, détendue.

2. Distracteur mandibulaire intraoral selon la revendication 1, **caractérisé en ce que** la pièce élastique (15) est une rondelle élastique du type Grower, ou une rondelle tronconique dite Belleville, ou un empilement de telles rondelles.

3. Distracteur mandibulaire intraoral selon la revendication 1, **caractérisé en ce que** la pièce élastique (15) est soit un ressort, soit un diaphragme, soit un cylindre compressible, par exemple en élastomère.

4. Distracteur mandibulaire intraoral selon l'une quelconque des revendications 1 à 3, **caractérisé en ce que** l'axe (12) commandant la rotation de la vis sans fin (11) est équipé d'une tête de manoeuvre (21) logée dans un évidement (20) pratiqué dans la paroi externe (19) du palier (14) contre lequel la pièce élastique (15) vient en appui.

5. Distracteur mandibulaire intraoral selon la revendication 4, **caractérisé en ce que** la tête (21) équipant l'axe (12) de commande de la rotation de la vis sans fin (11) présente une fente (22), ou deux fentes disposées en croix, ou un trou borgne (22) à six pans permettant la manoeuvre en rotation de ladite tête au moyen d'un tournevis ou d'une clé Allen.

6. Distracteur mandibulaire intraoral selon l'une quelconque des revendications 1 à 5, **caractérisé en ce que** ses deux moyens d'ancrage (1, 2) et respectivement son chariot mobile (10) comportent des pattes (3, 4) et respectivement des ailes (23) percées chacune de deux orifices (5, 24 respectivement) pour le passage de vis ou de broches garantissant leur fixation dans le corps mandibulaire et, respectivement, dans le segment osseux à distracter.

7. Distracteur mandibulaire intraoral selon la revendication 6, **caractérisé en ce que** les moyens (23) de fixation du chariot mobile (10) dans le segment osseux à distracter ont la forme d'un I, d'un Y ou d'une croix de Saint-André.

8. Distracteur mandibulaire intraoral selon l'une quelconque des revendications 1 à 7, **caractérisé en ce que** son rail cylindrique fileté (6) présente deux méplats (26) diamétralement opposés, le chariot mobile (10) guidé à coulissement sur ledit rail ayant la forme d'une cage dont les deux parois (27, 28) parallèles à l'axe longitudinal du rail, réunies à leurs deux extrémités par l'un et l'autre des deux paliers (13, 14) supportant l'axe (12) de la vis sans fin (11), sont espacées d'une distance très légèrement supérieure à l'épaisseur dudit rail (6) prise entre ses deux méplats (26).

9. Distracteur mandibulaire intraoral selon la revendication 8, **caractérisé en ce que** son rail cylindrique fileté (6) présente un troisième méplat en regard du fond (32) du chariot mobile (10).

10. Distracteur mandibulaire intraoral selon l'une quelconque des revendications 1 à 9, **caractérisé en ce que** son rail (6) est rectiligne.

11. Distracteur mandibulaire intraoral selon l'une quelconque des revendications 1 à 9, **caractérisé en ce que** son rail (6) forme au moins une courbe.

12. Distracteur mandibulaire intraoral selon la revendication 11, **caractérisé en ce que** la ou les différentes courbures du rail (6) appartiennent à un plan unique, lequel plan est perpendiculaire aux méplats (26) du rail afin de préserver le filetage au voisinage des fibres neutres (29) dudit rail.

13. Distracteur mandibulaire intraoral selon l'une quelconque des revendications 1 à 12, **caractérisé en ce que** chacun de ses éléments constitutifs est réalisé en un matériau biocompatible, par exemple en titane ou en alliage de titane.

## Patentansprüche

1. Intraorale Unterkiefer-Distraktionsvorrichtung nach Maß, die für die Rekonstruktion eines fehlenden oder schadhaften Knochensegments infolge einer Tumorresektion, eines Traumas, einer Knochenfehlstellung oder einer Knochenhypoplasie mittels Distraktionsosteogenese bestimmt ist, wobei die Distraktionsvorrichtung zwei Verankerungsmittel (1, 2) umfasst, die in dem Unterkieferkörper an fixen Verankerungspunkten befestigt sind, die sich beiderseits des knöchernen Kallus befinden, **dadurch gekennzeichnet, dass** sie umfasst:
a) eine zylindrische Gewindeschiene (6), die die beiden Verankerungsmittel (1, 2) verbindet,
b) einen beweglichen Schlitten (10), der gleitend auf der Schiene (6) geführt wird und auf dem zu distrahierenden Knochensegment befestigt ist, wobei der bewegliche Schlitten mit einer Schnecke (11) versehen ist, deren Gewinde zu jenem der Schiene komplementär ist und mit jenem zusammenwirkt,
c) eine Achse (12), die mit der Schnecke (11) verbunden ist und die Drehung derselben steuert, wobei die Achse im Wesentlichen parallel zur Schiene (6) ist und von zwei Lagern (13, 14) getragen wird, die auf dem beweglichen Schlitten (10) beiderseits der Schnecke vorgesehen sind,
d) mindestens ein elastisches Stück (15), das von der Achse (12) durchquert wird und einerseits an einem Ende (16) der Schnecke (11) und andererseits an der Innenwand (18) des Lagers (14) anliegt, welches dem Ende (16) gegenüberliegt, wobei der Abstand zwischen den Innenwänden (17, 18) der beiden Lager (13, 14) des beweglichen Schlittens (10) gleich der Länge der Schnecke (11), erhöht um die Höhe des elastischen Stücks (15) ist, wenn dieses in Ruhe und entspannt ist.

2. Intraorale Unterkiefer-Distraktionsvorrichtung nach Anspruch 1, **dadurch gekennzeichnet, dass** das elastische Stück (15) eine elastische Scheibe des Typs Grower oder eine kegelstumpfartige so genannte Belleville-Scheibe oder eine Stapelung solcher Scheiben ist.

3. Intraorale Unterkiefer-Distraktionsvorrichtung nach Anspruch 1, **dadurch gekennzeichnet, dass** das elastische Stück (15) entweder eine Feder oder eine Membran oder ein komprimierbarer Zylinder beispielsweise aus Elastomer ist.

4. Intraorale Unterkiefer-Distraktionsvorrichtung nach einem der Ansprüche 1 bis 3, **dadurch gekennzeichnet, dass** die Achse (12), die die Drehung der Schnecke (11) steuert, mit einem Betätigungskopf (21) ausgestattet ist, der in einer Ausnehmung (20) angeordnet ist, die in der Außenwand (19) des Lagers (14) vorgesehen ist, an dem das elastische Stück (15) anliegt.

5. Intraorale Unterkiefer-Distraktionsvorrichtung nach Anspruch 4, **dadurch gekennzeichnet, dass** der Kopf (21), mit dem die Achse (12) zur Steuerung der Drehung der Schnecke (11) versehen ist, einen Schlitz (22) oder zwei kreuzweise angeordnete Schlitze oder ein Grundloch (22) mit sechs Flächen aufweist, die die Drehbetätigung des Kopfes mit Hilfe eines Schraubenschlüssels oder eines Allen-Schlüssels ermöglichen.

6. Intraorale Unterkiefer-Distraktionsvorrichtung nach einem der Ansprüche 1 bis 5, **dadurch gekennzeichnet, dass** ihre beiden Verankerungsmittel (1, 2) und jeweils ihr beweglicher Schlitten (10) Laschen (3, 4) bzw. Flügel (23) umfassen, die jeweils mit zwei Öffnungen (5 bzw. 24) für die Durchführung von Schrauben oder Spindeln versehen sind, die ihre Befestigung in dem Unterkieferkörper bzw. in dem zu dehnenden Knochensegment gewährleisten.

7. Intraorale Unterkiefer-Distraktionsvorrichtung nach Anspruch 6, **dadurch gekennzeichnet, dass** die Mittel (23) zur Befestigung des beweglichen Schlittens (10) in dem zu distrahierenden Knochensegment die Form eines I, eine Y oder eines Andreaskreuzes haben.

8. Intraorale Unterkiefer-Distraktionsvorrichtung nach einem der Ansprüche 1 bis 7, **dadurch gekennzeichnet, dass** ihre zylindrische Gewindeschiene (6) zwei diametral gegenüberliegende Abflachungen (26) aufweist, wobei der bewegliche Schlitten (10), der gleitend auf der Schiene geführt wird, die Form eines Käfigs hat, dessen beiden Wände (27, 28) parallel zur Längsachse der Schiene, die an ihren beiden Enden durch das eine und das andere der beiden Lager (13, 14), die die Achse (12) der Schnecke (11) tragen, verbunden sind, um einen etwas größeren Abstand als die Dicke der Schiene (6) zwischen ihren Abflachungen (26) beabstandet sind.

9. Intraorale Unterkiefer-Distraktionsvorrichtung nach Anspruch 8, **dadurch gekennzeichnet, dass** ihre zylindrische Gewindeschiene (6) eine dritte Abflachung gegenüber dem Boden (32) des beweglichen Schlittens (10) aufweist.

10. Intraorale Unterkiefer-Distraktionsvorrichtung nach einem der Ansprüche 1 bis 9, **dadurch gekennzeichnet, dass** ihre Schiene (6) gerade ist.

11. Intraorale Unterkiefer-Distraktionsvorrichtung nach einem der Ansprüche 1 bis 9, **dadurch gekennzeichnet, dass** ihre Schiene (6) mindestens eine Krümmung bildet.

12. Intraorale Unterkiefer-Distraktionsvorrichtung nach Anspruch 11, **dadurch gekennzeichnet**, das die eine oder die verschiedenen Krümmungen der Schiene (6) einer einzigen Ebene angehören, wobei die Ebene zu den Abflachungen (26) der Schiene senkrecht ist, um das Gewinde in der Nähe der neutralen Fasern (29) der Schiene zu halten.

13. Intraorale Unterkiefer-Distraktionsvorrichtung nach einem der Ansprüche 1 bis 12, **dadurch gekennzeichnet, dass** jeder ihrer Bestandteile aus einem biokompatiblen Material, beispielsweise Titan oder einer Titanlegierung, hergestellt ist.

## Claims

1. Customised intraoral jaw distractor which is intended for the reconstruction by means of osteogenic distraction of a missing or defective bone segment following a tumour resection, a trauma, a bone deformation or a bone hypoplasia, the distractor, of the type comprising two anchoring means (1, 2) which are fixed in the jaw body at fixed anchoring locations, located at one side and the other of the bone callus, being **characterised in that** it comprises:
a) a threaded cylindrical rail (6) which connects the two anchoring means (1, 2),
b) a movable carriage (10) which is guided in a sliding manner on the rail (6) and which is fixed to the bone segment to be distracted, the movable carriage being provided with an endless screw (11) whose thread complements that of the rail and cooperates therewith,
c) a shaft (12) which is fixedly joined to the endless screw (11) and which controls the rotation thereof, the shaft being substantially parallel with the rail (6) and being supported by two bearings (13, 14) which are arranged on the movable carriage (10), at one side and the other of the endless screw,
d) at least one resilient component (15) through which the shaft (12) extends and which is in abutment, on the one hand, against an end (16) of the endless screw (11) and, on the other hand, against the inner wall (18) of the bearing (14) which is opposite the end (16), the distance separating the internal walls (17, 18) of the two bearings (13, 14) of the movable carriage (10) being equal to the length of the endless screw (11) increased by the height of the resilient component (15) when it is in the relaxed rest state.

2. Intraoral jaw distractor according to claim 1, **characterised in that** the resilient component (15) is a resilient washer of the Grower type, or a frustoconical washer referred to as a Belleville washer, or a stack of such washers.

3. Intraoral jaw distractor according to claim 1, **characterised in that** the resilient component (15) is a spring, a diaphragm or a compressible cylinder, for example, of elastomer material.

4. Intraoral jaw distractor according to any one of claims 1 to 3, **characterised in that** the shaft (12) which controls the rotation of the endless screw (11) is provided with a handling head (21) which is accommodated in a recess (20) formed in the outer wall (19) of the bearing (14) against which the resilient component (15) is in abutment.

5. Intraoral jaw distractor according to claim 4, **characterised in that** the head (21) with which the shaft (12) for controlling the rotation of the endless screw (11) is provided has an aperture (22), or two apertures which are arranged in the form of a cross, or a blind hole (22) having six faces which enable the head to be operated in terms of rotation using a screwdriver or an Allen key.

6. Intraoral jaw distractor according to any one of claims 1 to 5, **characterised in that** the two anchoring means (1, 2) and the movable carriage (10) thereof, respectively, comprise lugs (3, 4) and wings (23) through each of which two holes (5, 24, respectively) extend for the passage of screws or spindles which ensure the fixing thereof in the jaw body and the bone segment to be distracted, respectively.

7. Intraoral jaw distractor according to claim 6, **characterised in that** the means (23) for fixing the movable carriage (10) in the bone segment to be distracted are in the form of an I, a Y or a St. Andrew's cross.

8. Intraoral jaw distractor according to any one of claims 1 to 7, **characterised in that** the threaded cylindrical rail (6) thereof has two flattened portions (26) which are diametrically opposed, the movable carriage (10) which is guided in terms of sliding on the rail having the shape of a cage whose two walls (27, 28), which are parallel with the longitudinal axis of the rail and which are connected at the two ends thereof by one and the other of the two bearings (13, 14) which support the shaft (12) of the endless screw (11), are spaced apart by a distance which is very slightly greater than the thickness of the rail (6) which is engaged between the two flattened portions (26) thereof.

9. Intraoral jaw distractor according to claim 8, **characterised in that** the threaded cylindrical rail (6) thereof has a third flattened portion opposite the base (32) of the movable carriage (10).

10. Intraoral jaw distractor according to any one of claims 1 to 9, **characterised in that** the rail (6) thereof is rectilinear.

11. Intraoral jaw distractor according to any one of claims 1 to 9, **characterised in that** the rail (6) thereof forms at least one curve.

12. Intraoral jaw distractor according to claim 11, **characterised in that** the different curvature(s) of the rail (6) belong to a single plane which is perpendicular to the flattened portions (26) of the rail in order to maintain the threading in the region of the neutral axes (29) of the rail.

13. Intraoral jaw distractor according to any one of claims 1 to 12, **characterised in that** each of the constituent elements thereof is produced from a biocompatible material, for example, of titanium or titanium alloy.
